# EUROPÄISCHE PATENTSCHRIFT

(11) **EP 4 281 031 B1**
(45) Veröffentlichungstag und Bekanntmachung des Hinweises auf die Patenterteilung: **03.09.2025**
(21) Anmeldenummer: 22701895.9
(22) Anmeldetag: 13.01.2022
(51) Int. Cl.: A61K 8/06, A61K 8/19, A61K 8/34, A61K 8/365, A61K 8/37, A61K 8/39, A61K 8/42, A61K 8/55, A61K 8/67, A61K 8/92, A61Q 19/00

(54) **MINERALÖLFREIE KOSMETISCHE W/O-EMULSION**
MINERAL OIL-FREE COSMETIC W/O EMULSION
ÉMULSION COSMÉTIQUE E/H SANS HUILE MINÉRALE

(30) Priorität: 20.01.2021 DE 102021000268
(43) Veröffentlichungstag der Anmeldung: 29.11.2023
(73) Patentinhaber: Beiersdorf AG, 22529 Hamburg (DE)
(72) Erfinder: TIMM, Sebastian, 22765 Hamburg (DE); KOCH, Petra, 22457 Hamburg (DE); XU, Yang, 20249 Hamburg (DE)
(86) Internationale Anmeldenummer: PCT/EP2022/050606
(87) Internationale Veröffentlichungsnummer: WO 2022/157061

(56) Entgegenhaltungen:
- EP-A2- 0 965 331
- EP-B1- 2 931 210
- WO-A1-2022/002978
- WO-A2-2012/167905
- US-A1- 2015 011 654
- US-B2- 10 195 123

## Beschreibung

Die vorliegende Erfindung betrifft eine kosmetische Wasser-in-Öl Emulsion (W/O-Emulsion) enthaltend Triglyceride, wobei die Zubereitung frei ist von Mineralöl, Paraffinwachs, mikrokristallinem Wachs, Schellackwachs und Polyethylenwachsen, Polyacrylaten, quervernetzten Acrylat/C10-C30 Alkylacrylat Polymeren, Vinylpyrrolidon/Hexadecen-Copolymeren, 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-di-phenylacrylat (INCI: Octocrylen), Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, dadurch gekennzeichnet, dass die Zubereitung Diisostearoyl Polyglyceryl-3 Dimer Dilinoleate in einer Konzentration von 1,5 bis 2,5 Gewichts-%, bezogen auf das Gesamtgewicht der Zubereitung, enthält.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Hautpflegeprodukte, in der Regel Crémes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Neben der Reinigung und Pflege der Haut haben Kosmetika auch eine ästhetische Aufgabe. Sie sollen das äußere Erscheinungsbild des Anwenders entsprechend den jeweiligen kulturellen Vorstellungen "verbessern". Kosmetika erfüllen damit eine psychologisch-soziale Funktion, da sie die (optische) Attraktivität der Anwender erhöhen.

In jüngerer Zeit gibt es einen verstärkten Trend hin zu "natürlichen" Kosmetika, deren Inhaltsstoffe möglichst nicht mehr aus Erdölprodukten stammen oder chemisch synthetisiert sein sollen. Dabei stellt die Suche nach alternativen Inhaltsstoffen, welche diese Kriterien erfüllen, die Produktentwickler vor besondere Herausforderungen. Denn der Austausch der bekannten Inhaltstoffe wie Mineralöle, Silikonöle, Polyacrylate wird praktisch immer mit Nachteilen im Hinblick auf die Anwendung der Produkte erkauft. Die Zubereitungen werden sensorisch unattraktiv, was sich beispielsweise beim Verteilen der Zubereitung auf der Haut und dem mangelnden Einzugsvermögen unangenehm bemerkbar macht. EP 0 965 331 A offenbart Wasser-in-Öl-Emulsionen, die
- (a) eines Gehaltes an Wasser und gegebenenfalls wasserlöslichen Substanzen von insgesamt mindestens 85 Gew.-%, bezogen auf das Gesamtgewicht der Zubereitungen und eines Gehaltes an Lipiden, Emulgatoren und lipophilen Bestandteilen von insgesamt höchstens 15 Gew.-%.
- (b) wenigstens eine grenzflächenaktive Substanz, gewählt aus der Gruppe der Alkylmethiconcopolyole und/oder Alkyl-Dimethiconcopolyole,
- (c) eine oder mehrere Lipidkomponenten, welche die Lipidphase der Emulsion umfassen, enthalten,
- (d) wobei das Gewichtsverhältnis von (b) zu (c) aus dem Bereich 0,10 bis 0,25 gewählt wird.

Es war daher die Aufgabe der vorliegenden Erfindung eine kosmetische Emulsion (insbesondere eine W/O-Emulsion) zu entwickeln, die frei ist von Mineralölen, Silikonölen und Polyacrylaten, welche die gleichen sensorischen Eigenschaften aufweist wie herkömmliche Kosmetika, welche diese Inhaltsstoffe enthalten. Diese Zubereitungen sollten idealerweise "vegan" sein, das heißt ohne Inhaltsstoffe tierischen Ursprunges auskommen. Darüber hinaus wurde angestrebt, eine Zubereitung zu entwickeln, deren Inhaltsstoffe pflanzlichen Ursprungs sind, wobei unter pflanzlichen Ursprung auch solche Stoffe zu verstehen sind, deren Ausgangsstoff aus Pflanzen stammt und die anschließend einen oder mehrere Derivatisierungsschritte (z.B. Hydrolyse, Veresterung) durchlaufen.

Kosmetische Wasser-in-Öl Emulsionen (W/O Emulsionen) werden insbesondere als Grundlage für Zubereitungen verwendet, die der Hautbefeuchtung und der Rückfettung der Haut dienen. Hierbei dient die Emulsions-Grundlage neben ihrer Rolle als Lipid-Spender auch als Träger für Hautbefeuchtungsmittel wie Glycerin und Panthenol (auch Dexpanthenol genannt), die sich in der Wasserphase der Emulsion befinden. Um kosmetisch/dermatologisch wirksam zu werden, müssen diese Stoffe dann durch das Verdampfen des Trägerstoffes Wasser aus der Wasserphase freigesetzt werden. Die Verdunstungsgeschwindigkeit des Wassers ist also in der Regel der geschwindigkeitsbestimmende Schritt für den Wirkungseintritt der in der Wasserphase enthaltenden Wirkstoffe.

Es war daher die Aufgabe der vorliegenden Erfindung, eine Wasser-in-Öl Emulsion (W/O Emulsion) zu entwickeln, bei der die in der Wasserphase enthaltenden Wirkstoffe (insbesondere Glycerin und Panthenol) schneller freigesetzt werden.

Insbesondere war es die Aufgabe der vorliegenden Erfindung, die Verdunstungsgeschwindigkeit des Wassers aus der Emulsion zu erhöhen.

Überraschend gelöst werden die Aufgaben durch eine kosmetische Wasser-in-Öl Emulsion (W/O-Emulsion) enthaltend Triglyceride, wobei die Zubereitung frei ist von Mineralöl, Paraffinwachs, mikrokristallinem Wachs, Schellackwachs und Polyethylenwachsen, Polyacrylaten, quervernetzten Acrylat/C10-C30 Alkylacrylat Polymeren, Vinylpyrrolidon/Hexadecen-Copolymeren, 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen), Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, dadurch gekennzeichnet, dass Zubereitung Diisostearoyl Polyglyceryl-3 Dimer Dilinoleate in einer Konzentration von 1,5 bis 2,5 Gewichts-%, bezogen auf das Gesamtgewicht der Zubereitung, enthält.

Die Lösung der Aufgabe war insbesondere deshalb für den Fachmann nicht naheliegend, weil er davon ausgehen musste, dass der Wasseraustritt und damit die Verdunstungsgeschwindigkeit durch die Bildung von Wasserstoffbrücken zu den Sauerstoffatomen der Triglyceridester stärker behindert wird im Vergleich zu den Mineralölen, Paraffinwachsen und mikrokristallinen Wachsen herkömmlicher W/O-Emulsionen. Dies ist jedoch nicht der Fall.

Es ist erfindungsgemäß vorteilhaft, wenn die Menge der Wasserphase 35 bis 75 Gewichts-% und die Menge der Ölphase 25 bis 65 Gewichts-%, bezogen auf die Gesamtmenge der Zubereitung, beträgt.

Dabei ist es erfindungsgemäß bevorzugt, wenn die Zubereitung frei ist von aliphatischen und aromatischen Kohlenwasserstoffen.

Erfindungsgemäß vorteilhafte Ausführungsformen der vorliegenden Erfindung sind dadurch gekennzeichnet, dass die Zubereitung zusätzlich geradkettige und/oder verzweigte Fettalkohole enthält.

Dabei ist es erfindungsgemäß bevorzugt, wenn als geradkettig und/oder verzweigte Fettalkohole Octyldodecanol, Cetylalkohol und/oder Stearylalkohol eingesetzt werden. Ebenso ist es erfindungsgemäß bevorzugt, diese geradkettig und/oder verzweigten Fettalkohole in einer Gesamtkonzentration von 0,3 bis 2,5 Gewichts-%, bezogen auf das Gesamtgewicht der Zubereitung, einzusetzen.

Diisostearoyl Polyglyceryl-3 Dimer Dilinoleate kann beispielsweise unter dem Handelsnamen Isolan PDI bei der Firma Evonik erworben werden.

Es ist erfindungsgemäß vorteilhaft, wenn der Gehalt an Triglyceriden in der Emulsion von 10 bis 30 Gewichts-%, bezogen auf das Gesamtgewicht der Zubereitung, beträgt. Erfindungsgemäß bevorzugt ist dabei der Konzentrationsbereich von 20 bis 27 Gewichts-%, bezogen auf das Gesamtgewicht der Zubereitung.

Vorteilhafte Ausführungsformen der vorliegenden Erfindung sind ferner dadurch gekennzeichnet, dass Zubereitung Lecithin und/oder Glycerin enthält. Die erfindungsgemäß bevorzugte Einsatzkonzentration für Lecithin beträgt dabei 0,0005 bis 0,1 Gewichts-%, bezogen auf das Gesamtgewicht der Zubereitung.

Die erfindungsgemäß bevorzugte Einsatzkonzentration für Glycerin beträgt dabei 2 bis 6 Gewichts-%, bezogen auf das Gesamtgewicht der Zubereitung.

Darüber hinaus ist es erfindungsgemäß vorteilhaft, wenn die erfindungsgemäße Zubereitung einen oder mehrere Ester gewählt aus der Gruppe der Verbindungen Ascorbylpalmitat, Tocopherylacetat, Cetylpalmitat enthält.

Die erfindungsgemäß bevorzugte Einsatzkonzentration für Ascobylpalmitat beträgt dabei 0,0003 bis 0,1 Gewichts-%, bezogen auf das Gesamtgewicht der Zubereitung.

Die erfindungsgemäß bevorzugte Einsatzkonzentration für Tocopherylacetat beträgt dabei 0,0001 bis 0,1 Gewichts-%, bezogen auf das Gesamtgewicht der Zubereitung.

Die erfindungsgemäß bevorzugte Einsatzkonzentration für Cetylpalmitat beträgt dabei 0,005 bis 1,0 Gewichts-%, bezogen auf das Gesamtgewicht der Zubereitung.

Es ist erfindungsgemäß von Vorteil, wenn Zubereitung ein oder mehrere Salze gewählt aus der Gruppe der Verbindungen Magnesiumsulfat, Natriumanisat, Natriumcitrat enthält. Dabei kann Natriumcitrat, was auch in Form seiner freien Säure (Zitronensäure) eingesetzt werden, was erfindungsgemäß bevorzugt ist.

Die erfindungsgemäß bevorzugte Einsatzkonzentration für Magnesiumsulfat beträgt dabei 0,3 bis 1,5 Gewichts-%, bezogen auf das Gesamtgewicht der Zubereitung.

Die erfindungsgemäß bevorzugte Einsatzkonzentration für Natriumanisat beträgt dabei 0,002 bis 0,9 Gewichts-%, bezogen auf das Gesamtgewicht der Zubereitung.

Die erfindungsgemäß bevorzugte Einsatzkonzentration für Zitronensäure bzw. Natriumcitrat beträgt dabei 0,001 bis 0,8 Gewichts-%, bezogen auf das Gesamtgewicht der Zubereitung. Erfindungsgemäß vorteilhafte Ausführungsformen der vorliegenden Erfindung sind dadurch gekennzeichnet, dass Zubereitung Phenoxyethanol und/oder Panthenol enthält.

Die erfindungsgemäß bevorzugte Einsatzkonzentration für Phenoxyethanol beträgt dabei 0,01 bis 0,7 Gewichts-%, bezogen auf das Gesamtgewicht der Zubereitung.

Die erfindungsgemäß bevorzugte Einsatzkonzentration für Panthenol beträgt dabei 0,05 bis 0,94 Gewichts-%, bezogen auf das Gesamtgewicht der Zubereitung.

Erfindungsgemäß besonders bevorzugt ist der Einsatz einer Kombination aus Panthenol, insbesondere D-Panthenol und Glycerin.

Die erfindungsgemäß vorteilhaften Ausführungsformen der vorliegenden Erfindung sind dadurch gekennzeichnet, dass die Triglyceride gewählt werden aus Mischungen von hydriertem Rapsöl, Rapsöl, Capryl/Caprinsäure Triglyceriden, Sonnenblumenöl, hydrierten Kokosglyceriden, hydriertem Rizinusöl und Sheabutter, wobei die Mischung bevorzugt aus mehreren oder allen dieser Triglycerid-Quellen besteht.

Enthält die erfindungsgemäße Zubereitung hydriertes Rapsöl (INCI: Hydrogenated Rapeseed Oil), so wird dieses erfindungsgemäß bevorzugt in einer Konzentration von 4 bis 15 Gewichts-%, bezogen auf das Gesamtgewicht der Zubereitung eingesetzt.

Enthält die erfindungsgemäße Zubereitung Capryl/Caprinsäure Triglyceride (INCI: Caprylic/Capric Triglycerides), so wird dieses erfindungsgemäß bevorzugt in einer Konzentration von 5,1 bis 10 Gewichts-%, bezogen auf das Gesamtgewicht der Zubereitung eingesetzt.

Enthält die erfindungsgemäße Zubereitung Rapsöl (INCI: Brassica Campestris Seed Oil), so wird dieses erfindungsgemäß bevorzugt in einer Konzentration von 2 bis 4 Gewichts-%, bezogen auf das Gesamtgewicht der Zubereitung eingesetzt.

Enthält die erfindungsgemäße Zubereitung Sonnenblumenöl (insbesondere solches mit der INCI Heliathus Annuus Hybrid Oil), so wird dieses erfindungsgemäß bevorzugt in einer Konzentration von 1,6 bis 5 Gewichts-%, bezogen auf das Gesamtgewicht der Zubereitung eingesetzt.

Die erfindungsgemäße W/O-Emulsion liegt üblicherweise in Form einer Creme vor.

### Vergleichsversuch

Es wurden die folgenden Zubereitungen hergestellt und die Geschwindigkeit des Wasserverlustes gravimetrisch bestimmt.

| Inhaltsstoffe (INCI) | Creme OP 1483 (Gew.-%) | Creme OP 1702 (Gew.-%) |
|---|---|---|
| Aqua | Ad. 100 | Ad. 100 |
| Hydrogenated Rapeseed Oil, Brassica Campestris Seed Oil, Hydrogenated Coco Glycerides, Butyrospermum Parkii Oil | | 13,5 |
| Paraffinum Liquidum, Cera Microcristallina, Tocopheryl Acetate, BHT | 13,5 | |
| Dilsostearoyl Polyglyceryl-3 Dimer Dilinoleate | 2 | 2 |
| Caprylic/Capric Triglycerides, Helianthus Annuus Hybrid Oil, Hydrogenated Castor Oil* | 10,76 | 10,76 |
| Cetearyl Alcohol, Octyldodecanol* | 0,92 | 0,92 |
| Cetyl Palmitate | 0,3 | 0,3 |
| Glycerin | 4 | 4 |
| Panthenol | 0,1 | 0,1 |
| Magnesium Sulfate | 0,7 | 0,7 |
| Lecithin, Ascorbyl Palmitate, Phenoxyethanol, Pantolactone, Sodium Anisate, Citric Acid, Parfum* | 0,43 | 0,43 |

| | | |
|---|---|---|
| *= in beiden Rezepturen wurde die identische Mischung eingesetzt. | | |

### Durchführung:

Es wurden pro Emulsion jeweils die Rückseiten von 10 Petrischalen mit Creme bedeckt. Hierbei wurde das Eincremen der Haut simuliert (kreisende Bewegungen). Vor Applikation wurde das Leergewicht der jeweiligen Petrischale bestimmt. Anschließend erfolgte eine Bestimmung des Gewichtes nach Creme Applikation. Aus der Differenz der beiden Messwerte ergibt sich das Gewicht der applizierten Creme.

**Tabelle 1: Gewichtsbestimmung der mineralölhaltigen Creme**

| Creme OP 1483 | Gewicht (g) | Gewicht + Creme (g) | **Creme (g)** |
|---|---|---|---|
| 1 | 46,74 | 47,32 | **0,58** |
| 2 | 46,06 | 46,56 | **0,5** |
| 3 | 46,1 | 46,75 | **0,65** |
| 4 | 46,08 | 46,72 | **0,64** |
| 5 | 46,4 | 47,06 | **0,66** |
| 6 | 46,35 | 46,96 | **0,61** |
| 7 | 46,09 | 46,79 | **0,7** |
| 8 | 46,46 | 47,23 | **0,77** |
| 9 | 46,23 | 47,04 | **0,81** |
| 10 | 46,79 | 47,49 | **0,7** |

**Tabelle 2: Gewichtsbestimmung der mineralölfreien Creme**

| Creme OP 1702 | Gewicht (g) | Gewicht + Creme (g) | **Creme (g)** |
|---|---|---|---|
| 1 | 46 | 46,53 | **0,53** |
| 2 | 46,02 | 46,78 | **0,76** |
| 3 | 46,71 | 47,19 | **0,48** |
| 4 | 45,85 | 46,31 | **0,46** |
| 5 | 45,84 | 46,44 | **0,6** |
| 6 | 46,03 | 46,55 | **0,52** |
| 7 | 46,55 | 47,25 | **0,7** |
| 8 | 45,97 | 46,72 | **0,75** |
| 9 | 46,1 | 46,73 | **0,63** |
| 10 | 46,56 | 47,27 | **0,71** |

Nachdem die Petrischalen mit der jeweiligen Creme bedeckt wurden, erfolgte eine Gewichtsbestimmung zu den folgenden Zeitpunkten:
- 30 Minuten
- 60 Minuten
- 90 Minuten
- 180 Minuten
- 300 Minuten

In den nachfolgenden Tabellen sind die jeweiligen Gewichte der Cremes zu den oben genannten Messzeitpunkten abgebildet.

**Tabelle 3: Gewicht der mineralölhaltigen Proben zu den unterschiedlichen Messzeitpunkten**

| Creme OP 1483 | Creme (g) 0 min | Creme (g) 30 min | Creme (g) 60 min | Creme (g) 90 min | Creme (g) 180 min | Creme (g) 300 min |
|---|---|---|---|---|---|---|
| 1 | 0,58 | 0,56 | 0,55 | 0,55 | 0,52 | 0,49 |
| 2 | 0,5 | 0,48 | 0,47 | 0,47 | 0,45 | 0,42 |
| 3 | 0,65 | 0,63 | 0,64 | 0,63 | 0,6 | 0,58 |
| 4 | 0,64 | 0,63 | 0,62 | 0,61 | 0,59 | 0,56 |
| 5 | 0,66 | 0,65 | 0,64 | 0,64 | 0,62 | 0,59 |
| 6 | 0,61 | 0,59 | 0,58 | 0,59 | 0,57 | 0,54 |
| 7 | 0,7 | 0,68 | 0,67 | 0,68 | 0,68 | 0,64 |
| 8 | 0,77 | 0,75 | 0,76 | 0,75 | 0,74 | 0,71 |
| 9 | 0,81 | 0,79 | 0,78 | 0,78 | 0,76 | 0,73 |
| 10 | 0,7 | 0,76 | 0,67 | 0,66 | 0,65 | 0,62 |

**Tabelle 4: Gewicht der mineralölfreien Proben zu den unterschiedlichen Messzeitpunkten**

| Creme OP 1702 | Creme (g) 0 min | Creme (g) 30 min | Creme (g) 60 min | Creme (g) 90 min | Creme (g) 180 min | Creme (g) 300 min |
|---|---|---|---|---|---|---|
| 1 | 0,53 | 0,43 | 0,37 | 0,35 | 0,27 | 0,21 |
| 2 | 0,76 | 0,61 | 0,58 | 0,54 | 0,46 | 0,37 |
| 3 | 0,48 | 0,35 | 0,3 | 0,27 | 0,21 | 0,18 |
| 4 | 0,46 | 0,32 | 0,3 | 0,27 | 0,21 | 0,17 |
| 5 | 0,6 | 0,46 | 0,41 | 0,38 | 0,3 | 0,24 |
| 6 | 0,52 | 0,39 | 0,34 | 0,3 | 0,23 | 0,18 |
| 7 | 0,7 | 0,55 | 0,51 | 0,47 | 0,39 | 0,31 |
| 8 | 0,75 | 0,59 | 0,55 | 0,52 | 0,44 | 0,34 |
| 9 | 0,63 | 0,5 | 0,44 | 0,42 | 0,33 | 0,25 |
| 10 | 0,71 | 0,58 | 0,52 | 0,49 | 0,4 | 0,31 |

Wie man an Tabelle 3 erkennt, verlieren die mineralölhaltigen Muster nur sehr geringfügig an Gewicht. Dies ist bei der mineralölfreien Creme nicht der Fall (Tabelle 4). Hier ist bereits innerhalb der ersten 30 Minuten eine deutliche Gewichtsabnahme, sprich ein Wasserverlust zu erkennen. In den zwei nachfolgenden Tabellen 5 und 6 sind die jeweiligen prozentualen Gewichtsverluste abgebildet.

**Tabelle 5: Gewichtsverlust in % der mineralölhaltigen Ceme**

| Creme OP 1483 | Gewichtsverlu st (%) 30 min | Gewichtsverlu st (%) 60 min | Gewichtsverlust (%) 90 min | Gewichtsverlust (%) 180 min | Gewichtsverlust (g) 300 min |
|---|---|---|---|---|---|
| 1 | 3 | 5 | 5 | 10 | 16 |
| 2 | 4 | 6 | 6 | 10 | 16 |
| 3 | 3 | 2 | 3 | 8 | 11 |
| 4 | 2 | 3 | 5 | 8 | 12 |
| 5 | 2 | 3 | 3 | 6 | 11 |
| 6 | 3 | 5 | 3 | 7 | 11 |
| 7 | 3 | 4 | 3 | 3 | 9 |
| 8 | 3 | 1 | 3 | 4 | 8 |
| 9 | 2 | 4 | 4 | 6 | 10 |
| 10 | 3 | 4 | 4 | 7 | 11 |

Nach 30 Minuten haben die Muster im Schnitt nur 3% Gewicht verloren. Nach 90 Minuten sind es gerade mal 4%.

**Tabelle 6: Gewichtsverlust in % der mineralölfreien Creme**

| Creme OP 1483 | Creme (g) 30 min | Creme (g) 60 min | Creme (g) 90 min | Creme (g) 180 min | Creme (g) 300 min |
|---|---|---|---|---|---|
| 1 | 19 | 30 | 34 | 49 | 60 |
| 2 | 20 | 24 | 29 | 39 | 51 |
| 3 | 27 | 38 | 44 | 56 | 62 |
| 4 | 30 | 35 | 41 | 54 | 63 |
| 5 | 23 | 32 | 37 | 50 | 60 |
| 6 | 25 | 35 | 42 | 56 | 65 |
| 7 | 21 | 27 | 33 | 44 | 56 |
| 8 | 21 | 27 | 31 | 41 | 55 |
| 9 | 21 | 30 | 33 | 48 | 60 |
| 10 | 18 | 27 | 31 | 44 | 56 |
| Mittelwert | 23 | 30 | 35 | 84 | 59 |

Nach 30 Minuten haben die mineralölfreien Muster bereits 23% an Gewicht, sprich Wasser verloren.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| Inhaltsstoffe (INCI) | Beispiel 1 | Beispiel 2 | Beispiel 3 | Beispiel 4 |
|---|---|---|---|---|
| Aqua | Ad. 100 | Ad. 100 | Ad. 100 | Ad. 100 |
| Hydrogenated Rapeseed Oil | 8,47 | 6,12 | 4,15 | 7,10 |
| Brassica Campestris Seed Oil | 2,55 | 2,28 | 3,22 | 3,32 |
| Hydrogenated Coco Glycerides | 1,64 | 1,53 | 1,85 | 1,27 |
| Butyrospermum Parkii Oil | 1,43 | 1,94 | 1,75 | 1,79 |
| Diisostearoyl Polyglyceryl-3 Dimer Dilinoleate | 1,60 | 1,80 | 2,20 | 2,00 |
| Caprylic/Capric Triglycerides | 5,50 | 9,50 | 7,00 | 5,70 |
| Helianthus Annuus Hybrid Oil | 4,00 | 2,00 | 4,80 | 4,75 |
| Hydrogenated Castor Oil | 0,40 | 0,40 | 0,40 | 0,40 |
| Cetearyl Alcohol | 0,33 | 0,70 | 0,50 | 0,30 |
| Octyldodecanol | 0,66 | 0,80 | 1,00 | 0,75 |
| Cetyl Palmitate | 0,90 | 0,50 | 0,70 | 0,10 |
| Lecithin | 0,0055 | 0,0073 | 0,0033 | 0,0068 |
| Ascorbyl Palmiate | 0,0045 | 0,0033 | 0,0077 | 0,0041 |
| Parfum | 0,17 | 0,20 | 0,22 | 0,15 |
| Glycerin | 3,00 | 4,50 | 5,50 | 2,50 |
| Panthenol | 0,80 | 0,50 | 0,40 | 0,20 |
| Magnesium Sulfate | 0,50 | 0,60 | 0,80 | 0,70 |
| Phenoxyethanol | 0,20 | 0,15 | 0,10 | 0,02 |
| Pantolactone | 0,0213 | 0,013 | 0,0106 | 0,005 |
| Sodium Anisate | 0,10 | 0,15 | 0,20 | 0,06 |
| Citric Acid | 0,04 | 0,06 | 0,08 | 0,02 |

## Patentansprüche

1. Kosmetische Wasser-in-Öl Emulsion (W/O-Emulsion) enthaltend Triglyceride, wobei die Zubereitung frei ist von Mineralöl, Paraffinwachs, mikrokristallinem Wachs, Schellackwachs und Polyethylenwachsen, Polyacrylaten, quervernetzten Acrylat/C10-C30 Alkylacrylat Polymeren, Vinylpyrrolidon/Hexadecen-Copolymeren, 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen), Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, **dadurch gekennzeichnet, dass** die Zubereitung Diisostearoyl Polyglyceryl-3 Dimer Dilinoleate in einer Konzentration von 1,5 bis 2,5 Gewichts-%, bezogen auf das Gesamtgewicht der Zubereitung, enthält.

2. Kosmetische W/O-Emulsion nach Anspruch 1, **dadurch gekennzeichnet, dass** die Zubereitung frei ist von aliphatischen und aromatischen Kohlenwasserstoffen.

3. Kosmetische W/O-Emulsion nach einem der vorhergehenden Ansprüche, **dadurch gekennzeichnet, dass** die Zubereitung zusätzlich geradkettige und/oder verzweigte Fettalkohole enthält.

4. Kosmetische W/O-Emulsion nach Anspruch 3, **dadurch gekennzeichnet, dass** als geradkettig und/oder verzweigte Fettalkohole Octyldodecanol, Cetylalkohol und/oder Stearylalkohol eingesetzt werden.

5. Kosmetische W/O-Emulsion nach einem der vorhergehenden Ansprüche, **dadurch gekennzeichnet, dass** der Gehalt an Triglyceriden in der Emulsion 10 bis 30 Gewichts-%, bezogen auf das Gesamtgewicht der Zubereitung, beträgt.

6. Kosmetische W/O-Emulsion nach einem der vorhergehenden Ansprüche, **dadurch gekennzeichnet, dass** Zubereitung Lecithin und/oder Glycerin enthält.

7. Kosmetische W/O-Emulsion nach einem der vorhergehenden Ansprüche, **dadurch gekennzeichnet, dass** Zubereitung einen oder mehrere Ester gewählt aus der Gruppe der Verbindungen Ascorbylpalmitat, Tocopherylacetat, Cetylpalmitat enthält.

8. Kosmetische W/O-Emulsion nach einem der vorhergehenden Ansprüche, **dadurch gekennzeichnet, dass** Zubereitung einen oder mehrere Salze gewählt aus der Gruppe der Verbindungen Magnesiumsulfat, Natriumanisat, Natriumcitrat enthält.

9. Kosmetische W/O-Emulsion nach einem der vorhergehenden Ansprüche, **dadurch gekennzeichnet, dass** Zubereitung Phenoxyethanol und/oder Panthenol enthält.

10. Kosmetische W/O-Emulsion nach einem der vorhergehenden Ansprüche, **dadurch gekennzeichnet, dass** die Triglyceride gewählt werden aus Mischungen von hydriertem Rapsöl, Rapsöl, Capryl/Caprinsäure Triglyceriden, Sonnenblumenöl, hydrierten Kokosglyceriden, hydriertem Rizinusöl und Sheabutter, wobei die Mischung bevorzugt aus mehreren oder allen dieser Triglycerid-Quellen besteht.

## Claims

1. Cosmetic water-in-oil emulsion (W/O emulsion) comprising triglycerides, the preparation being free of mineral oil, paraffin wax, microcrystalline wax, shellac wax and polyethylene waxes, polyacrylates, crosslinked acrylate/C10-C30 alkyl acrylate polymers, vinylpyrrolidone/hexadecene copolymers, 3-(4-methylbenzylidene)camphor, 2-hydroxy-4-methoxybenzophenone (INCI: Oxybenzone), 2-ethylhexyl 4-methoxycinnamate (INCI: Octylmethoxycinnamate), ethylhexyl 2-cyano-3,3-diphenylacrylate (INCI: Octocrylene), parabens (especially ethyl-, propyl-, and butylparaben), methylisothiazolinone, chloromethylisothiazolinone and DMDM hydantoin, polyethylene glycol ethers or polyethylene glycol esters, **characterized in that** the preparation contains diisostearoyl polyglyceryl-3 dimer dilinoleate in a concentration of 1.5% to 2.5% by weight based on the total weight of the preparation.

2. Cosmetic W/O emulsion according to Claim 1, **characterized in that** that the preparation is free of aliphatic and aromatic hydrocarbons.

3. Cosmetic W/O emulsion according to either of the preceding claims, **characterized in that** the preparation additionally comprises straight-chain and/or branched fatty alcohols.

4. Cosmetic W/O emulsion according to Claim 3, **characterized in that** octyldodecanol, cetyl alcohol and/or stearyl alcohol are employed as straight-chain and/or branched fatty alcohols.

5. Cosmetic W/O emulsion according to any of the preceding claims, **characterized in that** the content of triglycerides in the emulsion is from 10% to 30% by weight based on the total weight of the preparation.

6. Cosmetic W/O emulsion according to any of the preceding claims, **characterized in that** preparation comprises lecithin and/or glycerol.

7. Cosmetic W/O emulsion according to any of the preceding claims, **characterized in that** preparation comprises one or more esters selected from the group of compounds ascorbyl palmitate, tocopheryl acetate, and cetyl palmitate.

8. Cosmetic W/O emulsion according to any of the preceding claims, **characterized in that** preparation comprises one or more salts selected from the group of compounds magnesium sulfate, sodium anisate, and sodium citrate.

9. Cosmetic W/O emulsion according to any of the preceding claims, **characterized in that** preparation comprises phenoxyethanol and/or panthenol.

10. Cosmetic W/O emulsion according to any of the preceding claims, **characterized in that** the triglycerides are selected from mixtures of hydrogenated rapeseed oil, rapeseed oil, caprylic/capric acid triglycerides, sunflower oil, hydrogenated coconut glycerides, hydrogenated castor oil, and shea butter, the mixture preferably consisting of a plurality or all of these triglyceride sources.

## Revendications

1. Émulsion cosmétique eau-dans-huile (émulsion E/H) contenant des triglycérides, la préparation étant exempte d'huile minérale, de cire de paraffine, de cire microcristalline, de cire de gomme laque et de cires de polyéthylène, de polyacrylates, de polymères réticulés d'acrylate/acrylate de C10-C30-alkyle, de copolymères de vinylpyrrolidone/hexadécène, de 3-(4-méthylbenzylidène)-camphre, de 2-hydroxy-4-méthoxybenzophénone (INCI : Oxybenzone), d'ester 2-éthylhexylique de l'acide 4-méthoxycinnamique (INCI : Octylmethoxycinnamate), de 2-cyano-3,3-diphénylacrylate d'éthylhexyle (INCI : Octocrylene), de parabènes (en particulier de méthylparabène, de propylparabène et de butylparabène), de méthylisothiazolinone, de chlorométhylisothiazolinone et de DMDM-hydantoïne, de polyéthylèneglycoléthers ou d'esters de polyéthylèneglycol, **caractérisée en ce que** la préparation contient du dilinoléate de dimère de diisostéaroyl-polyglycéryl-3 en une concentration de 1,5 à 2,5% en poids, par rapport au poids total de la préparation.

2. Émulsion cosmétique E/H selon la revendication 1, **caractérisée en ce que** la préparation est exempte d'hydrocarbures aliphatiques et aromatiques.

3. Émulsion cosmétique E/H selon l'une des revendications précédentes, **caractérisée en ce que** la préparation contient en outre des alcools gras linéaires et/ou ramifiés.

4. Émulsion cosmétique E/H selon la revendication 3, **caractérisée en ce que** l'octyldodécanol, l'alcool cétylique et/ou l'alcool stéarylique sont utilisés en tant qu'alcools gras linéaires et/ou ramifiés.

5. Émulsion cosmétique E/H selon l'une des revendications précédentes, **caractérisée en ce que** la teneur en triglycérides dans l'émulsion représente 10 à 30% en poids par rapport au poids total de la préparation.

6. Émulsion cosmétique E/H selon l'une des revendications précédentes, **caractérisée en ce que** la préparation contient de la lécithine et/ou du glycérol.

7. Émulsion cosmétique E/H selon l'une des revendications précédentes, **caractérisée en ce que** la préparation contient un ou plusieurs esters choisis dans le groupe des composés palmitate d'ascorbyle, acétate de tocophéryle, palmitate de cétyle.

8. Émulsion cosmétique E/H selon l'une des revendications précédentes, **caractérisée en ce que** la préparation contient un ou plusieurs sels choisis dans le groupe des composés sulfate de magnésium, anisate de sodium, citrate de sodium.

9. Émulsion cosmétique E/H selon l'une des revendications précédentes, **caractérisée en ce que** la préparation contient du phénoxyéthanol et/ou du panthénol.

10. Émulsion cosmétique E/H selon l'une des revendications précédentes, **caractérisée en ce que** les triglycérides sont choisis parmi les mélanges d'huile de colza hydrogénée, d'huile de colza, de triglycérides d'acide caprylique/caprique, d'huile de tournesol, de glycérides de coco hydrogénés, d'huile de ricin hydrogénée et de beurre de karité, le mélange étant de préférence constitué par plusieurs de ces sources de triglycérides ou de toutes ces sources de triglycérides.
